(19) Europäisches Patentamt
European Patent Office
Office européen des brevets

(11) Publication number: **0 130 862**
**B1**

(12) # EUROPEAN PATENT SPECIFICATION

(45) Date of publication of patent specification: **17.08.88**

(51) Int. Cl.$^4$: **C 07 H 15/22,** A 61 K 31/70

(21) Application number: **84401075.1**

(22) Date of filing: **24.05.84**

(54) **N-methanesulfonic acid derivatives of 3-demethoxy-istamycin B and production thereof.**

(30) Priority: **25.05.83 JP 90752/83**

(43) Date of publication of application:
**09.01.85 Bulletin 85/02**

(45) Publication of the grant of the patent:
**17.08.88 Bulletin 88/33**

(84) Designated Contracting States:
**DE FR GB IT**

(56) References cited:
**EP-A-0 108 563**
**FR-A-2 521 142**
**GB-A-2 083 464**
**US-A-4 567 165**

(73) Proprietor: **ZAIDAN HOJIN BISEIBUTSU KAGAKU KENKYU KAI**
**14-23, Kami Osaki 3-chome**
**Shinagawa-ku Tokyo (JP)**

(72) Inventor: **Umezawa, Hamao**
**23, Toyotama Kita 4-chome Nerima-ku**
**Tokyo (JP)**
Inventor: **Kondo, Shinichi**
**1157-1, Ichigao-cho Midori-ku**
**Yokohama-shi Kanagawa-ken (JP)**

(74) Representative: **Nony, Michel et al**
**Cabinet Nony 29, rue Cambacérès**
**F-75008 Paris (FR)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 130 862 B1

**Description**

Summary of the invention

This invention relates to less toxic derivatives of 3-demethoxyistamycin B which are new compounds or substances useful for therapeutic treatment of bacterial infections. More particularly, this invention relates to new N-methanesulfonic acid derivatives of 3-demethoxyistamycin B and also to a process for the production of said derivatives.

Background of the invention

Istamycin $B_0$ is an aminoglycosidic antibiotic which was discovered by the present inventors and is produced in the culture broth of *Streptomyces tenjimariensis* (see Japanese patent application prepublication "Kokai" No. 43295/81; U.K. Patent 2048855; and U.S. Patent No: 4296106). 3-Demethoxyistamycin B is a new semi-synthetic aminoglycosidic antibiotic which was synthesized by us from istamycin $B_0$ and exhibits a high antibacterial activity against a wide range of gram-negative and gram-positive bacteria, including *Pseudomonas aeruginosa* (see Japanese Patent application No. 193436/82; EPC patent application No. 108.563 and U.S. patent 4,479,943. 3-Demethoxyistamycin B is represented by the formula

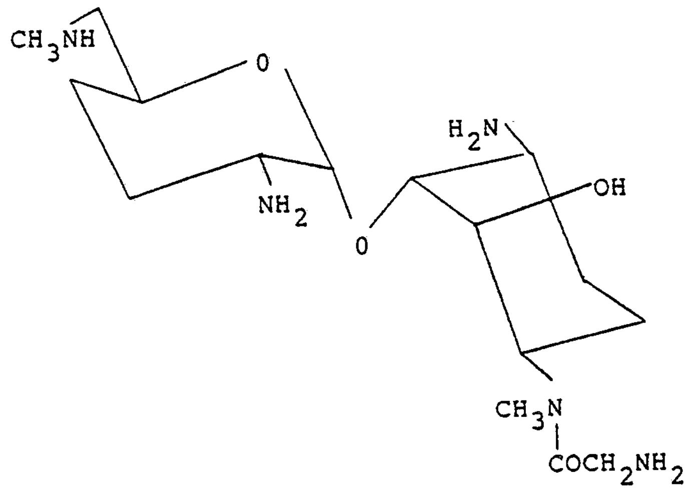

(I)

It is known that a few of aminoglycosidic antibiotic substances are converted into an N-methanesulfonic acid derivatives thereof by N-sulfomethylation of some or all of the amino group(s) present in the antibiotic molecule, and that the N-methanesulfonic acid derivatives so produced exhibit a lower toxicity than the parent antibiotic. Examples are N-methanesulfonic acid derivatives of kanamcycin A (Journal of Antibiotics, A *14,* page 170 (1961)). Besides, it has been found by the present inventors than an N-methanesulfonic acid derivative of 3',4'-dideoxykanamycin B can be synthesized by reaction of 3',4'-dideoxykanamycin B, an aldehyde and sulfurous acid or an alkali metal hydrogen sulfite with each other, and that this N-methanesulfonic acid derivative is of lower toxicity than the parent 3',4'-dideoxykanamycin B and hence is valuable for therapeutic treatment of bacterial infections (see Japanese patent application prepublication "Kokai" No. 39653/77; U.K. Patent No. 1,507,118; U.S. Patent No. 4.091,202). It has also been found by us that an N-methanesulfonic acid derivative of istamycin A or B and that of 3-0-demethylistamycin B may be obtained as new less toxic substances having useful antibacterial activity (see Japenese patent application prepublication "Kokai" No. 40496/82; published U.K. patent application GB 2,083,464) for the former and Japanese patent application prepublication "Kokai" No. 128395/83) for the latter).

The discoveries mentioned above are solely related to N-methanesulfonic acid derivatives of kanamycin, istamycins A and B and 3-0-demethylistamycin B and are not extensively applicable to those of all the aminoglycosidic antibiotics.

An object of this invention is to provide a new antibiotic derivative of 3-demethoxyistamycin B which, we have found, retains useful antibacterial activity of 3-demethoxyistamycin B but exhibits a lower toxicity than that of 3-demethoxyistamycin B itself. The other object is to provide a process for the preparation of such new antibiotic derivative of 3-demethoxyistamycin B. Another objects of this invention will be clear from the following descriptions.

As a result of our research, we have now found that as new compounds or substances, N-methanesulfonic acid derivatives of 3-demethoxyistamycin B can be synthesized by reaction of 3-demethoxyistamycin B of the above formula (I) or a partial acid addition salt thereof with paraformaldehyde and also with sulfurous acid or an alkali or alkaline earth metal hydrogen sulfite or ammonium hydrogen sulfite of the formula:

$$MHSO_3 \qquad (IV)$$

wherein M is a hydrogen atom, an alkali metal, alkaline earth metal atom or ammonium cation. We have confirmed that these N-methanesulfonic acid derivatives of 3-demethoxyistamycin B are of remarkably lower toxicity than 3-demethoxyistamycin B itself. 3-Demethoxyistamycin B contains three amino groups and one methylamino group per molecule as will be clear from the above formula (I), and it has been found that the new N-methanesulfonic acid derivative of 3-demethoxyistamycin B so prepared is such one in which one, two, three or four groups of the aforesaid three amino groups and one methylamino group present in the molecule has or have been N-sulfomethylated, that is to say, substituted with a methanesulfonate group of the formula:

$$—CH_2SO_3M \qquad (II)$$

wherein M represents a hydrogen atom, an ammonium cation, an alkali metal or an alkaline earth metal atom. The total number of the N-sulfomethylated amino and methylamino groups present in the resulting N-methanesulfonic acid derivative of 3-demethoxyistamycin B amounts to 1, 2, 3 or 4, depending upon the molar proportions of the paraformaldehyde and the sulfurous acid or sulfite compound employed for 1 molar proportion of 3-demethoxyistamycin B.

Detailed description of the invention

According to a first aspect of this invention, there is provided as a new compound or substance, an N-methanesulfonic acid derivative of 3-demethoxyistamycin B of the formula:

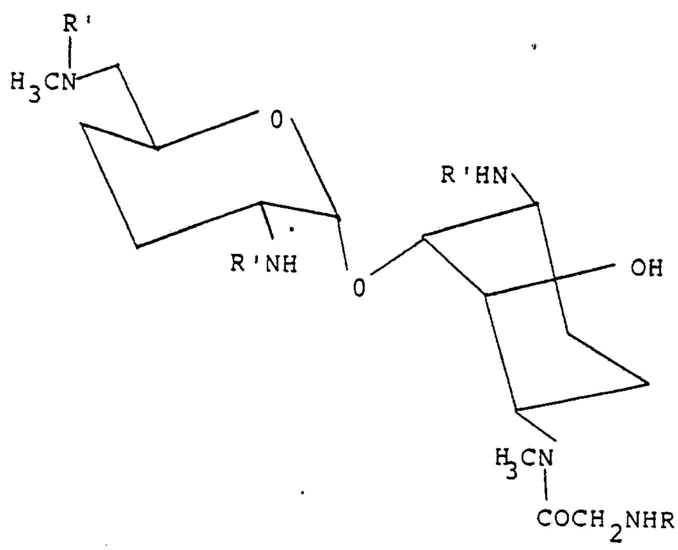

(III)

wherein one, two, three or four of the R′ groups denote(s) each a group $—CH_2SO_3M$ and the remaining other R′ group(s) denote(s) each a hydrogen atom and M is a hydrogen atom, an ammonium cation, an alkali metal atom or an alkaline earth metal atom.

According to a particular embodiment of the first aspect invention, there is provided an N-methanesulfonic acid derivative of 3-demethoxyistamycin B which is selected from (1) 3-demethoxyistamycin B-mono-N-methanesulfonic acid sodium salt, namely the compound of the formula (III) where one R′ group is a group $—CH_2SO_3M$, the remaining three R′ groups are each a hydrogen atom, and M is sodium; (2) 3-demethoxyistamycin B-di-N-methanesulfonic acid sodium salt, namely the compound of the formula (III) where two R′ groups are each a group $—CH_2SO_3M$, the remaining two R′ groups are each a hydrogen atom and M is sodium; (3) 3-demethoxyistamycin B-tri-N-methane sulfonic acid sodium salt, namely the compound of the formula (III) where three R′ groups are each a group $—CH_2SO_3M$, the remaining one R′ group is a hydrogen atom and M is sodium; and (4) 3-demethoxyistamycin B-tetra-N-methanesulfonic acid sodium salt, namely the compound of the formula (III) where all four R′ groups are each a group $—CH_2SO_3M$, and M is sodium.

Particular examples of the N-methanesulfonic acid derivative of 3-demethoxyistamycin B obtained according to this invention are listed below together with physicochemical properties thereof:—

(1) 3-Demethoxyistamycin B-mono-N-methanesulfonic acid sodium salt of the formula $C_{16}H_{32}N_5O_4(CH_2SO_3Na)$. This substance is in the form of a colorless powder which has no definite melting point, decomposes gradually above 210°C and shows a specific optical rotation $[\alpha]_D^{23} = +49°$ (c=l, water).

Elemental analysis:

Found: S 7.40%

Calcd.: S 6.74%

(2) 3-Demethoxyistamycin B-di-N-methanesulfonic acid sodium salt of the formula $C_{16}H_{31}N_5O_4(CH_2SO_3Na)_2$.

This substance is in the form of a colorless powder which has no definite melting point, decomposes gradually at 248°C and shows a specific optical rotation $[\alpha]_D^{23} = +47°$ (c=l, water).

Elemental analysis:
Found: S 11.12%
Calcd.: S 10.84%

(3) 3-Demethoxyistamycin B-tri-N-methanesulfonic acid sodium salt of the formula $C_{16}H_{30}N_5O_4(CH_2SO_3Na)_3$. This substance is in the form of a colorless powder which has no definite melting point, decomposes gradually at 238°C and shows a specific optical rotation $[\alpha]_D^{23} = +46°$ (c=l, water).

Elemental analysis:
Found: S 13.24%
Calcd.: S 13.59%

(4) 3-Demethoxyistamycin B-tetra-N-methanesulfonic acid sodium salt of the formula $C_{16}H_{29}N_5O_4(CH_2SO_3Na)_4$. This compound is also in the form of a colorless powder which has no definite melting point, decomposes gradually at 219°C and shows a specific optical rotation $[\alpha]_D^{23} = +45°$ (c=l, water).

Elemental analysis:
Found: S 14.54%
Calcd.: S 15.57%

Each of the above-mentioned particular 3-demethoxyistamycin B N-methanesulfonic acid derivatives is readily soluble in water but little soluble or insoluble in a lower alkanol such as methanol, ethanol and 1-butanol, tetrahydrofuran, dioxane and N,N-dimethylformamide.

The new compounds or substances of this invention exhibit high antibacterial activity against a variety of gram-negative and gram-positive bacteria, including *Pseudomanas aeruginosa,* as will be clear from the anti-bacterial spectra of them shown in Table 1 below, wherein there are set out the minimum inhibitory concentrations (mcg/ml) of 3-demethoxyistamycin B-mono-N-methanesulfonic acid sodium salt [abbreviated as Compound (1)]; 3-demethoxyistamycin B-di-N-methanesulfonic acid sodium salt [abbreviated as Compound (2)]; 3-demethoxyistamycin B-tri-N-methanesulfonic acid sodium salt [abbreviated as Compound (3)]; and 3-demethoxyistamycin B-tetra-N-methanesulfonic acid sodium salt [abbreviated as Compound (4)] of this invention against various bacteria which have been estimated according to a standard serial dilution method using Mueller-Hinton agar as the incubation medium, the incubation being made at 37°C for 17 hours. Minimum inhibitory concentrations (mcg/ml) of the parent 3-demethoxyistamycin B were also estimated in the same manner for the comparison purpose and are also shown in Table 1.

TABLE 1

| Test Microorganism | Minimum Inhibitory concentrations (mcg/ml) | | | | |
|---|---|---|---|---|---|
| | Compound (1) | Compound (2) | Compound (3) | Compound (4) | 3-Demethoxy-istamycin B (comparative) |
| Staphylococcus aureus 209P | 0.78 | 0.78 | 1.56 | 1.56 | <0.20 |
| " ApO1 | 1.56 | 0.78 | 0.78 | 1.56 | 0.39 |
| Escherichia coli K-12 | 1.56 | 1.56 | 1.56 | 1.56 | 0.78 |
| " " K-12 R5 | 6.25 | 3.13 | 3.13 | 6.25 | 1.56 |
| " " K-12 ML1629 | 1.56 | 1.56 | 1.56 | 3.13 | 0.78 |
| " " K-12 LA290 R55 | 3.13 | 1.56 | 1.56 | 3.13 | 1.56 |
| " " JR66/W677 | 6.25 | 3.13 | 3.13 | 6.25 | 0.78 |
| " " K-12 C600 R135 | 6.25 | 6.25 | 6.25 | 6.25 | 0.78 |
| " " JR225 | 1.56 | 1.56 | 1.56 | 1.56 | 0.78 |
| Serratia marcescens | 6.25 | 3.13 | 6.25 | 3.13 | 0.78 |
| Providencia Pv 16 | 3.13 | 1.56 | 3.13 | 1.56 | 0.78 |
| Pseudomonas aeruginosa A3 | 0.78 | 0.78 | 0.78 | 1.56 | 0.39 |
| " " H9 | 50 | 50 | 50 | 50 | 12.5 |
| " " TI-13 | 25 | 25 | 25 | 25 | 6.25 |
| " " GN315 | 100 | 100 | 50 | 25 | 6.25 |

The N-methanesulfonic acid derivatives of 3-demethoxyistamycin B according to this invention have a remarkably reduced acute toxicity, as compared with the parent 3-demethoxyistamycin B, notwithstanding that the former compounds or substances of this invention retain high antibacterial activity against various bacteria.

Acute toxicity of the various N-methanesulfonic acid derivatives of 3-demethoxyistamycin B of the invention has been determined by the following procedure:

A test compound or substance was disssolved in 0.25 ml of a physiological saline solution and the solution of the test compound or substance so prepared was intravenously administered into a series of mouse groups each consisting of six mice (ICR strain, adult female, body weight 20 g ± 0.5 g) as the test animal, so that the test compound or substance was given to each mouse. Acute toxicity of 3-demethoxyistamycin B was also estimated in the same manner as above for the comparison purpose. It was then observed that all mice survived for more than 14 days when the N-methanesulfonic acid derivatives of 3-demethoxyistamycin B were administered at a dosage of 500 mg/kg ($LD_{50}$ more than 500 mg/kg), whereas 3-demethoxyistamycin B (comparative) showed $LD_{50}$ of 100-200 mg/kg.

From the test results of Table 1 and of acute toxicity as mentioned above, it is evident that the new compounds or substances of the invention have remarkably reduced toxicity but retain usefully high antibacterial activity against various bacteria.

The new compounds or substances of the invention are effective in the treatment of bacterial infections when administered intramuscularly in the dosage range of 100 mg to 1,000 mg per day in divided doses twice to four times a day. Generally, the new compounds or substances may be administered orally, intraperitoneally, intavenously or intramuscularly using any pharmaceutical form known to the art for such administration and in a similar manner to kanamycins. Examples of pharmaceutical forms for oral administration are powders, capsules, tablets and syrup.

The new N-methanesulfonic acid derivatives of 3-demethoxyistamycin B of the above formula (III) according to the invention may be prepared by reaction of 3-demethoxyistamycin B, either in the form of the free base or a partially acid-addition salt thereof, with paraformaldehyde and sulfurous acid or a sulfite of the formula:

$$MHSO_3 \qquad (IV)$$

wherein M is a hydrogen atom, an ammonium cation, an alkali metal atom or an alkaline earth metal atom. The resulting N-methanesulfonic acid derivative contains a certain number of N-methanesulfonate group(s) which takes 1, 2, 3 or 4 depending upon the molar proportions of paraformaldehyde and the sulfurous acid or sulfite compound employed for 1 molar proportion of 3-demethoxyistamycin B. Paraformaldehyde and the sulfurous acid or sulfite may be reacted simultaneously with 3-demethoxyistamycin B, or alternatively either one of the paraformaldehyde reagent and the sulfurous acid or sulfite reagent may be first reacted with 3-demethylistamycin B before the resulting reaction product is reacted with the other reagent.

According to a second aspect of this invention, therefore, there is provided a process for the preparation of N-methanesulfonic acid derivative of 3-demethoxyistamycin B of the formula

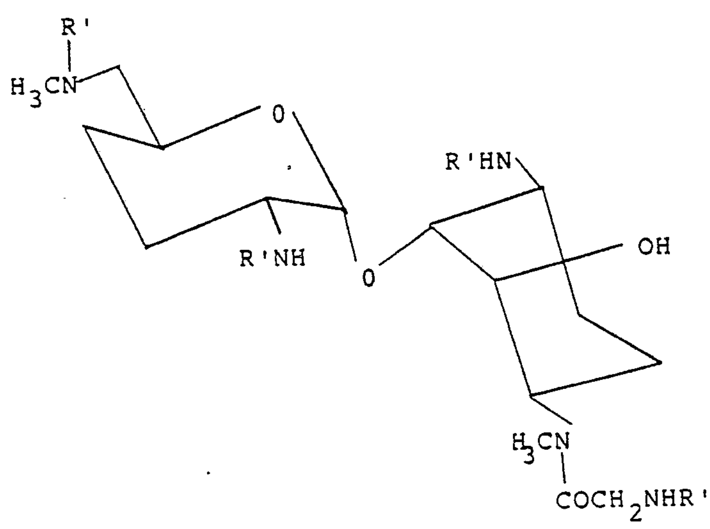

(III)

wherein one, two, three or four of the R′ groups denote(s) each a group $—CH_2SO_3M$ and the remaining other R′ group(s) denote(s) each a hydrogen atom, and M is a hydrogen atom, an ammonium cation, an alkali metal atom or an alkaline earth metal atom, which comprises reacting 3-demethoxyistamycin B or a

partial acid addition salt thereof with paraformaldehyde and sulfurous acid or a sulfite of the formula

$$MHSO_3 \quad (IV)$$

wherein M is as defined above.

When sulfurous acid of the above formula (V) where M is a hydrogen atom is used as one of the reagents in the process of the invention, it may conveniently be used in the form of gaseous sulfur dioxide. However, it it feasible, of course, to employ aqueous sulfurous acid. Instead of the sulfurous acid reagent, an alkali metal, alkaline earth metal or ammonium hydrogen sulfite may be used as an equivalent agent. Sodium hydrogen sulfite, potassium hydrogen sulfite, lithium hydrogen sulfite and ammonium hydrogen sulfite are suitable as the sulfite for the purpose of the invention.

In preparing the new compound of the above formula (III) according to the invention, either one of the paraformaldehyde reagent and the sulfurous acid or sulfite reagent of the formula (IV) may be first reacted with 3-demethoxyistamycin B. Thus, it is feasible to carry out the process in such a manner that the paraformaldehyde reagent is first reacted with 3-demethoxyistamycin B to produce the corresponding Schiff's base and the Schiff's base so formed is isolated and then reacted with the sulfurous acid or sulfite reagent to yield the desired 3-demethoxyistamycin B N-methanesulfonic acid derivative (III) as the final product.

Alternatively, it is possible to conduct the process in such a manner that the sulfurous acid or sulfite reagent is first reacted with 3-demethoxyistamycin B to convert the latter into the form of an acid-addition salt with sulfurous acid, which is subsequently reacted with the paraformaldehyde reagent to yield the desired N-methanesulfonic acid derivative (III) as the final product. Moreover, an adduct of both paraformaldehyde and (IV) such as sodium hydroxymethanesulfonate may also be used in the process of the invention. Namely, this adduct may be directly reacted with 3-demethoxyistamycin B to yield the desired N-methanesulfonic acid derivative (III) as the final product.

As will be clear from the above, the molar proportions of the paraformaldehyde reagent and the sulfurous acid or sulfite reagent to be interacted with 3-demethoxyistamycin B may vary from 1 molar to 4 molar proportions for 1 molar proportion of 3-demethoxyistamycin B. The N-methanesulfonic acid derivatives obtained as the final product by the process of the invention contain the methanesulfonate component at different contents depending on the molar proportions of the paraformaldehyde reagent and the sulfurous acid or sulfite reagent employed, but they usually contain one, two, three or four N-methanesulfonate groups per molecule of 3-demethoxyistamycin B.

Generally, the process may be carried out preferably in water as the reaction medium, but a small proportion of a lower alcohol such as methanol and ethanol may be added to the reaction medium. The process may readily be conducted at a temperature of 0 to 70°C usually for a reaction period of 0.5 to 24 hours.

For recovery of the final product from the reaction solution, it may be precipitated as a colorless deposit by adding thereto a volume of an organic solvent in which the desired product is sparingly soluble, such as a lower alcohol, e.g. methanol and ethanol, tetrahydrofuran, dioxane and N,N-dimethylformamide. The precipitate so formed is filtered out, washed with methanol or ethanol and then dried to afford the desired 3-demethoxyistamycin B N-methanesulfonic acid derivative (III) in a good yield.

As stated before, the new compounds or substances of the invention are effective in treatment of bacterial infections. According to a third aspect of the invention, therefore, there is provided an antibacterial pharmaceutical composition comprising an antibacterially effective amount of a 3-demethoxyistamycin B N-methanesulfonic acid derivative of the formula (III), in combination with a pharmaceutically acceptable carrier therefor.

The invention is now illustrated with reference to the following Examples which are in no way limitative for the invention.

## Example 1

3-Demethoxyistamycin B (free base) (100 mg; 0.28 millimoles) was dissolved in water (1.0 ml), and the resultant aqueous solution was admixed with sodium hydrogen sulfite (29 mg; 0.28 millimoles) and paraformaldehyde (8.5 mg; 0.28 millimoles). The mixture obtained was shaken at ambient temperature overnight for the reaction. Ethanol (10 ml) was added to the reaction solution to deposit a colorless precipitate. This precipitate was collected by filtration and dried under reduced pressure to a constant weight, affording 55 mg of a colorless powder of 3-demethoxyistamycin B-mono-N-methanesulfonic acid sodium salt which gradually decomposed above 210°C. $[\alpha]_D^{23} = +49°$ (c=l, water).

## Example 2

3-Demethoxyistamycin B (free base) (100 mg; 0.28 millimoles) was dissolved in water (1.0 ml), and the resultant aqueous solution was admixed with sodium hydrogen sulfite (58 mg; 0.56 millimoles) and paraformaldehyde (17 mg; 0.56 millimoles). The mixture obtained was shaken at ambient temperature overnight for the reaction. Ethanol (10 ml) was added to the reaction solution to deposit a colorless precipitate. This precipitate was collected by filtration and dried under reduced pressure to a constant weight, affording 95 mg of a colorless powder of 3-demethoxyistamycin B-di-N-methanesulfonic acid sodium salt which decomposed gradually at 248°C. $[\alpha]_D^{23} = +47°$ (c=l, water).

Example 3

3-Demethoxyistamycin B (free base) (100 mg; 0.28 millimoles) was dissolved in water (1.5 ml), and the resultant aqueous solution was admixed with sodium hydrogen sulfite (87 mg; 0.84 millimoles) and paraformaldehyde (25 mg; 0.84 millimoles). The mixture obtained was shaken at ambient temperature overnight for the reaction. Ethanol (12.5 ml) was added to the reaction solution to deposit a colorless precipitate. This precipitate was collected by filtration and dried under reduced pressure to a constant weight, affording 153 mg of a colorless powder of 3-demethoxyistamycin B-tri-N-methanesulfonic acid sodium salt which decomposed gradually at 238°C. $[\alpha]_D^{23} = +46°$ (c=l, water).

Example 4

3-Demethoxyistamycin B (free base) (100 mg; 0.28 millimoles) was dissolved in water (2.0 ml), and the resultant aqueous solution was admixed with sodium hydrogen sulfite (116 mg; 1.12 millimoles) and paraformaldehyde (34 mg; 1.12 millimoles). The mixture obtained was shaken at ambient temperature overnight for the reaction. Ethanol (15 ml) was added to the reaction solution to deposit a colorless precipitate. This precipitate was collected by filtration and dried under reduced pressure to a constant weight, affording 177 mg of a colorless powder of 3-demethoxyistamycin B-tetra-N-methanesulfonic acid sodium salt which decomposed gradually at 219°C. $[\alpha]_D^{23} = +45°$ (c=l, water).

**Claims**

1. An N-methanesulfonic acid derivative of 3-demethoxyistamycin B represented by the formula

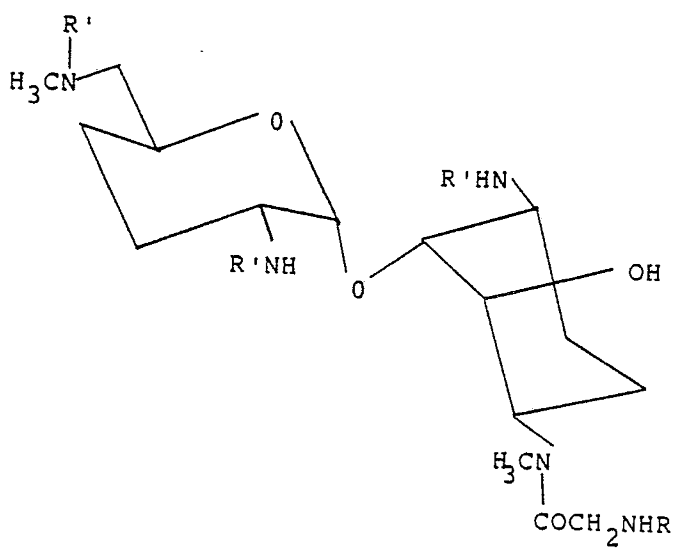

(III)

wherein one, two, three or four of the R′ groups denote(s) each a group $-CH_2SO_3M$ and the remaining other R′ group(s) denote(s) each a hydrogen atom, where M is a hydrogen atom, an ammonium cation, an alkali metal atom or an alkaline earth metal atom.

2. A derivative according to Claim 1 which is 3-demethoxyistamycin B-mono-N-methanesulfonic acid sodium salt, the compound of the formula (III) where one R′ group is a group $-CH_2SO_3M$ and the remaining three R′ groups are each a hydrogen atom; and M is sodium.

3. A derivative according to Claim 1 which is 3-demethoxyistamycin B-di-N-methanesulfonic acid sodium salt, the compound of the formula (III) where two R′ groups are each a group $-CH_2SO_3M$ and the remaining two R′ groups are each a hydrogen atom; and M is sodium.

4. A derivative according to Claim 1 which is 3-demethoxyistamycin B-tri-N-methanesulfonic acid sodium salt, the compound of the formula (III) where three R′ groups are each a group $-CH_2SO_3M$ and the remaining one R′ group is a hydrogen atom; and M is sodium.

5. A derivative according to Claim 1 which is 3-demethoxyistamycin B-tetra-N-methanesulfonic acid sodium salt, the compound of the formula (III) where all four R′ groups are each a group $-CH_2SO_3M$ and M is sodium.

6. A process for the preparation of an N-methanesulfonic acid derivative of 3-demethoxyistamycin B represented by the formula

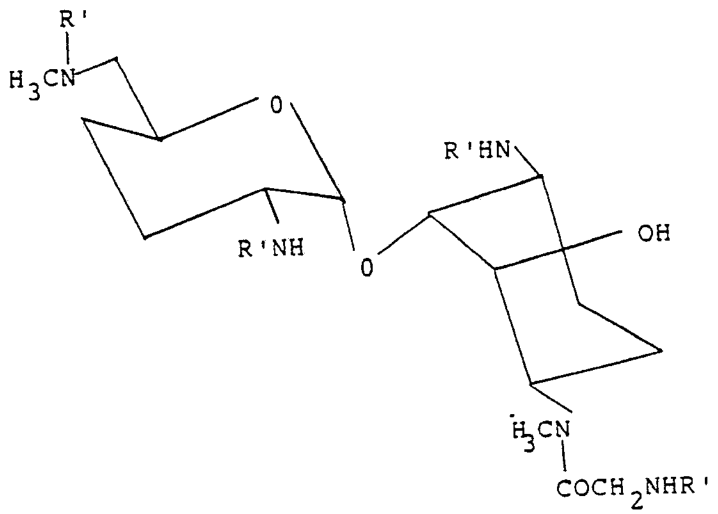

(III)

wherein one, two, three or four of the R′ groups denote(s) each a group $—CH_2SO_3M$ and the remaining other R′ group(s) denote(s) each a hydrogen atom, where M is a hydrogen atom, an ammonium cation, an alkali metal atom or an alkaline earth metal atom, which comprises reacting in water as the reaction medium 3-demethoxyistamycin B or a partial acid addition salt thereof with paraformaldehyde and sulfurous acid or a sulfite of the formula

$$MHSO_3 \qquad (IV)$$

wherein M is as defined above.

7. A process according to Claim 6 in which one molar proportion of 3-demethoxyistamycin B is reacted with one, two, three of four molar proportions of sodium hydrogen sulfite and with one, two, three or four molar proportions of paraformaldehyde at once in water at room temperature overnight, the molar proportions of sodium hydrogen sulfite and paraformaldehyde employed being equal to each other.

8. An antibacterial pharmaceutical composition comprising an antibacterially effective amount of the N-methanesulfonic acid derivative of 3-demethoxyistamycin B of formula (III) according to Claim 1, in combination with a pharmaceutically acceptable carrier therefor.

9. Use of the N-methanesulfonic acid derivative of 3-demethoxyistamycin B of formula (III) according to Claim 1 for the manufacture of a therapeutical composition to be used in the treatment of bacterial infections.

**Patentansprüche**

1. N-Methansulfonsäure-Derivat von 3-Demethoxyistamycin-B der allegemeinen Formel:

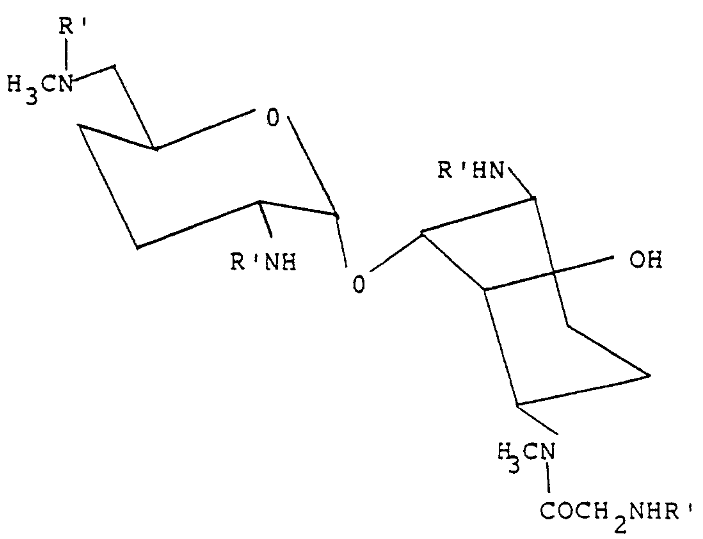

(III)

wobei eine, zwei, drei oder vier der R'-Gruppen eine —$CH_2SO_3M$-Gruppe und die verbleibende(n) andere(n) R'-Gruppe(n) ein Wasserstoffatom und M ein Wasserstoffatom, ein Ammoniumkation, ein Alkalimetallatom oder ein Erdalkalimetallatom bedeuten.

2. Derivat nach Anspruch 1, das das Natriumsalz von 3-Demethoxyistamycin B-mono-N-methansulfonsäure ist, wobei in der Verbindung gemäß der Formel (III) eine der R'-Gruppen eine $CH_2SO_3M$-Gruppe, die verbleibenden drei R'-Gruppen je ein Wasserstoffatom und M ein Natriumatom bedeuten.

3. Derivat nach Anspruch 1, das das Natriumsalz von 3-Demethoxyistamycin-B-di-N-methansulfonsäure ist, wobei in der Verbindung gemäß der Formel (III) zwei der R'-Gruppen je eine $CH_2SO_3M$-Gruppe, die verbleibenden zwei R'-Gruppen je ein Wasserstoffatom und M ein Natriumatom bedeuten.

4. Derivat nach Anspruch 1, das das Natriumsalz von 3-Demethoxyistamycin-B-tri-N-methansulfonsäure ist, wobei in der Verbindung gemäß der Formel (III) drei der R'-Gruppen je eine —$CH_2SO_3M$-Gruppe, die verbleibende R'-Gruppe ein Wasserstoffatom und M ein Natriumatom bedeuten.

5. Derivat nach Anspruch 1, das das Natriumsalz von 3-Demethoxyistamycin-B-tetramethansulfonsäure ist, wobei in der Verbindung gemäß der Formel (III) alle vier R'-Gruppen je eine $CH_2SO_3M$-Gruppe und M ein Natriumatom bedeuten.

6. Verfahren zur Herstellung von N-Methansulfonsäurederivaten von 3-Demethoxyistamycin-B der allgemeinen Formel:

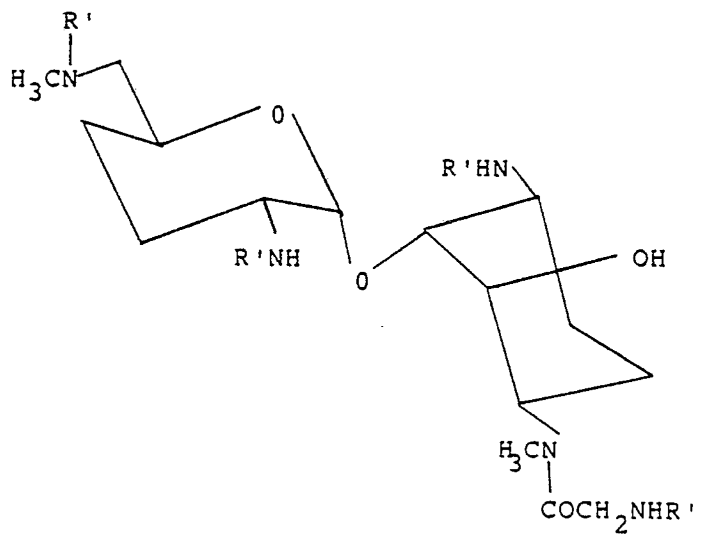

(III)

wobei eine, zwei, drei oder vier der R'-Gruppen eine —$CH_2SO_3M$-Gruppe und die verbleibende(n) andere(n) R'-Gruppe je ein Wasserstoffatom und M ein Wasserstoffatom, ein Ammoniumkation, ein Alkalimetallatom oder ein Erdalkalimetallatom bedeuten, dadurch gekennzeichnet daß in waßrigem Reaktionsmedium 3-Demethoxyistamycin-B oder ein teilweise saures Additionssalz davon mit Paraformaldehyd und schwefliger Säure oder einem Sulfit der Formel:

$$MHSO_3 \quad (IV)$$

umgesetzt wird, wobei M die vorstehend genannte Bedeutung hat.

7. Verfahren nach Anspruch 6, dadurch gekennzeichnet, daß das 3-Demethoxyistamycin-B in Wasser bei Raumtemperatur über Nacht auf einmal in einem molaren Verhältnis von eins zu eins, zwei, drei oder vier mit Natriumhydrogensulfit und in einem molaren Verhältnis von eins zu eins, zwei, drei oder vier mit Paraformaldehyd umgesetzt wird, wobei die molaren Verhältnisse für Natriumhydrogensulfit und Paraformaldehyd gleich sind.

8. Antibakterielle pharmazeutische Zubereitung, enthaltend eine antibakteriell wirksame Menge eines N-Methansulfonsäure-Derivates von 3-Demethoxyistamycin-B gemäß der Formel (III) nach Anspruch 1 in Kombination mit einem pharmzeutisch akzeptablen Träger.

9. Verwendung des N-Methansulfonsäure-Derivatives von 3-Demethoxyistamycin-B gemäß der allgemeinen Formel (III) nach Anspruch 1 für die Herstellung einer therapeutischen Zubereitung für die Behandlung von bakteriellen Infektionen.

**Revendications**

1. Dérivé d'acide N-méthanesulfonique de 3-déméthoxyistamycine B représenté par la formule:

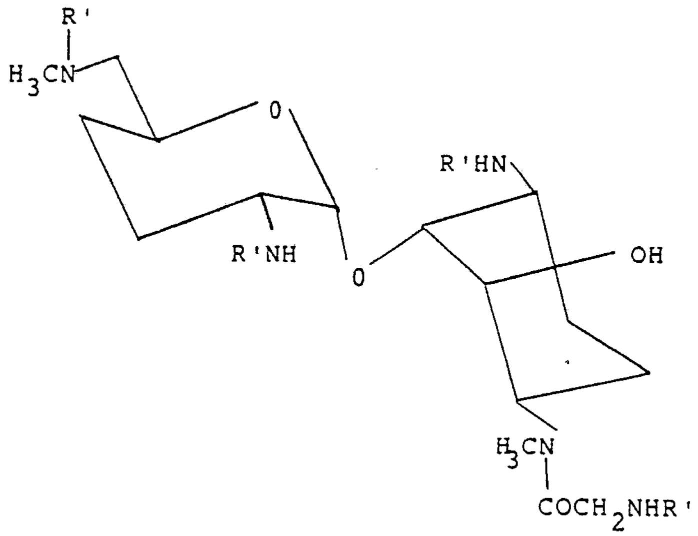

(III)

dans laquelle un, deux ou trois ou quatre des groupes R' représente(nt) chacun un groupe $-CH_2SO_3M$ et le ou les autre(s) groupe(s) R' représente(nt) chacun un atome d'hydrogène, où M est un atome d'hydrogène, un cation ammonium, un atome de métal alcalin ou un atome de métal alcalino-terreux.

2. Dérivé selon la revendication 1 caractérisé par le fait qu'il est le sel de sodium de l'acide 3-déméthoxyistamycine B-mono-N-méthanesulfonique, à savoir le composé de formule (III) dans laquelle un groupe R' est un groupe $-CH_2SO_3M$ et les trois autres groupes R' restants représentant chacun un atome d'hydrogène; et M est sodium.

3. Dérivé selon la revendication 1 caractérisé par le fait qu'il est le sel de sodium de l'acide 3-déméthoxyistamycine B-di-N-méthanesulfonique, à savoir le composé de formule (III) dans laquelle deux groupes R' représentent chacun un groupe $-CH_2SO_3M$ et les deux autres groupes R' restants représentant chacun un atome d'hydrogène; et M est sodium.

4. Dérivé selon la revendication 1 caractérisé par le fait qu'il est le sel de sodium de l'acide 3-déméthoxyistamycine B-tri-N-méthanesulfonique, à savoir le composé de formule (III) dans laquelle trois groupes R' représentent chacun un groupe $-CH_2SO_3M$ et le groupe R' restant étant un atome d'hydrogène; et M est sodium.

5. Dérivé selon la revendication 1 caractérisé par le fait qu'il est le sel de sodium de l'acide 3-déméthoxyistamycine B-tetra-N-méthanesulfonique, à savoir le composé de formule (III) dans lequel les quatre groupes R' représentent chacun un groupe $-CH_2SO_3M$ et M est sodium.

6. Procédé de préparation d'un dérivé d'acide N-méthanesulfonique de 3-déméthoxyistamycine B représenté par la formule:

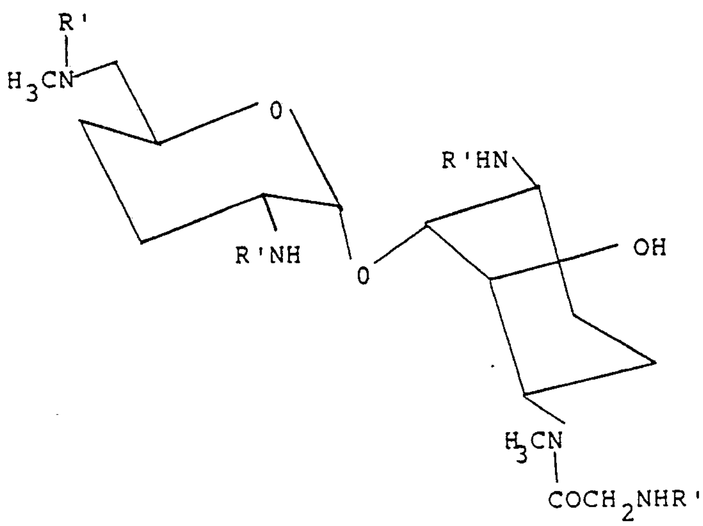

(III)

dans laquelle un, deux, trois ou quatre des groupes R′ représente(nt) chacun un groupe —$CH_2SO_3M$ et le ou les autre(s) groupe(s) R′ restant(s) représente(nt) chacun un atome d'hydrogène, où M est un atome d'hydrogène, un cation ammonium, un atome de métal alcalin ou un atome de métal alcalino-terreux, dans lequel on fait réagir dans l'eau, comme milieu réactionnel, la 3-déméthoxyistamycine B ou un de ses sels d'addition d'acide partielle avec du paraformaldéhyde et de l'acide sulfureux ou un sulfite de formule:

$$MHSO_3 \qquad (IV)$$

où M est tel que défini ci-dessus.

7. Procédé selon la revendication 6 dans lequel on fait réagir une proportion molaire de 3-déméthoxyistamycine B avec une, deux, trois, ou quatre proportions molaires de sulfite acide de sodium et avec une, deux, trois ou quatre proportions molaires de paraformaldéhyde en une seule fois dans l'eau à la température ambiante pendant la nuit, les proportions molaires de sulfite acide de sodium et de paraformaldéhyde employées étant égales entre elles.

8. Composition phamaceutique antibactérienne comprenant une quantité antibactériellement active du dérivé d'acide N-méthanesulfonique de 3-déméthoxyistamycine B de formule (III) selon la revendication 1, en mélange avec un support pharmaceutiquement acceptable.

9. Utilisation du dérivé d'acide N-méthanesulfonique de 3-déméthoxyistamycine B de formule (III) selon la revendication 1 pour la préparation d'une composition thérapeutique destinée à être utilisée dans le traitement d'infections bactériennes.